# EUROPÄISCHE PATENTSCHRIFT

(11) **EP 0 813 891 B1**
(45) Veröffentlichungstag und Bekanntmachung des Hinweises auf die Patenterteilung: **03.09.2003**
(21) Anmeldenummer: 97250186.0
(22) Anmeldetag: 16.06.1997
(51) Int. Cl.: A61N 1/37

(54) **Signaldetektor**
Signal detector
Détecteur de signaux

(30) Priorität: 18.06.1996 DE 19626353
(43) Veröffentlichungstag der Anmeldung: 29.12.1997
(73) Patentinhaber: BIOTRONIK Mess- und Therapiegeräte GmbH & Co Ingenieurbüro Berlin, 12359 Berlin (DE)
(72) Erfinder: Nigam, Indra B., Tigard, Oregon 97223 (US); Schaldach, Max, Prof. Dr., 91054 Erlangen (DE)
(74) Vertreter: Eisenführ, Speiser & Partner

(56) Entgegenhaltungen:
- EP-A- 0 350 160
- US-A- 4 708 144
- US-A- 5 117 824
- US-A- 5 339 820
- US-A- 5 513 644

## Beschreibung

Die Erfindung betrifft einen Signaldetektor zur Detektion von spezifischen Biosignalen, insbesondere zur Detektion von QRS-Komplexen in einem Elektrokardiogramm, gemäß dem Oberbegriff des Anspruchs 1. Ein soldier Signaldetektor ist aus US-A-5 513 644 bekannt.

Die Detektion spezifischer Biosignale in einem gestörten Signalzug ist ein Problem von hoher diagnostischer und therapeutischer Relevanz, beispielsweise in der Kardiologie und Neurologie. So ist die Erkennung der einzelnen Abschnitte eines Elektrokardiogramms - der vom Vorhof ausgehenden P-Welle, des von der Herzkammer ausgehenden QRS-Komplexes und ggfs. der ebenfalls aus der Kammer herrührenden T-Zacke - und die korrekte Bestimmung des Zeitabstandes von Signalabschnitten gleichen Ursprungs von zentraler Bedeutung für die Erkennung und erfolgreiche Behandlung von Herzrhythmusstörungen, speziell für die Klassifizierung von Tachyarrhythmien und die korrekte Funktion von Herzschrittmachern und Defibrillatoren.

Die Detektion des QRS-Komplexes in einem EKG-Signal erfolgt im einfachsten Fall durch Vergleich des EKG-Signals mit einem vorgegebenen Schwellwert, der oberhalb eines anzunehmenden Pegels von Signalen anderen Ursprungs liegt. Überschreitet das EKG-Signal den Schwellwert, so wird ein das Auftreten eines QRS-Komplexes anzeigendes Detektionssignal ausgegeben.

Problematisch hierbei ist, daß das Signalniveau des EKG-Signals Schwankungen unterliegt, was zu Fehldetektionen führen kann.

Dem Umstand, daß die QRS-Komplexe bei bestimmten Herzrhythmusstörungen (Arrhythmien) und im Zuge einer Schrittmachertherapie sehr unterschiedliche Amplituden aufweisen können, wird gemäß US 4 940 054 durch Programmierung dreier verschiedener Empfindlichkeits- oder Schwellwerte sowie eines vorbestimmten Ablaufes der Anwendung dieser verschiedenen Schwellwerte Rechnung getragen. Diese Lösung hat jedoch ein sehr begrenztes Anwendungspotential und ist insbesondere bei schwankendem Störsignalpegel kaum von Vorteil.

In DE 2 805 482 A1 wird ein QRS-Detektor mit automatischer Schwellwertanpassung beschrieben, bei dem die Nachweisschwelle der Amplitude der R-Wellen nachgeführt und nach Auftreten einer R-Welle jeweils mit durch die Bauelemente der analogen Schaltung vorbestimmter Zeitkonstante exponentiell abgesenkt wird. Auch diese Lösung ist wegen der bauelementseitigen Festlegung des Zeitverhaltens wenig flexibel.

In US 5 117 824 wird ein R-Wellen-Detektor beschrieben, bei dem die Nachweisschwelle automatisch der Amplitude der R-Wellen nachgeführt und jeweils vom Zeitpunkt des Auftretens einer R-Welle ab linear abgesenkt wird, bis die nächste R-Welle erscheint. Der Anfangs-Schwellwert wird dann neu auf einen vorbestimmten Anteil von deren Amplitude festgesetzt. Eine durch einen Schrittmacherimpuls evozierte R-Welle wird jedoch ignoriert und die lineare Absenkung für eine vorbestimmte Zeitspanne weitergeführt, um auch etwa folgende R-Wellen niedriger Amplitude detektieren zu können (wie sie bei schrittmacherinduzierten Tachyarrhythmien auftreten können). Auch diese Lösung ist wenig anpassungsfähig für verschiedene Nutz-/Störsignalkonstellationen.

Bei dem aus der US-PS 5 339 820 bekannte Signaldetektor ist eine Refraktärzeit vorgesehen und die Detektionsempfindlichkeit wird automatisch eingestellt. Der Signaldetektor startet bei einem Überschreiten einer vorgegebenen Anfangs-Nachweisschwelle (Detektionsereignis) eine Refraktärzeit, innerhalb derer keine weiteren Detektionsereignisse stattfinden können, um zu verhindern, daß ein einzelner QRS-Komplex fälschlich mehrfach detektiert wird. Darüber hinaus ermittelt der Signaldetektor während der Refraktärzeit die Amplitude des QRS-Komplexes. Danach wird der Schwellwert in Abhängigkeit von der zuvor ermittelten Amplitude des QRS-Komplexes auf einen neuen Wert (bevorzugt 75% der Amplitude) eingestellt. Anschließend wird der Schwellwert dann zeitgesteuert stufenweise abgesenkt. Das Absenken des Schwellwerts wird beendet, wenn entweder der nächste QRS-Komplex detektiert wird oder der Schwellwert einen programmierten unteren Grenzwert erreicht.

Oftmals betreffen die Schwankungen des Eingangssignals jedoch nicht oder nicht allein das spezifische Nutzsignal, sondern (auch) andere Signale, unter anderem Störsignale. In einem derartigen Fall kann es vorkommen, daß der vorbekannte Signaldetektor den Schwellwert unter den Pegel der unspezifischen Signale absenkt, was zu Fehldetektionen führt. Zudem ist ein Vorgehen, bei dem ausschließlich eine Absenkung der Nachweisschwelle vorgesehen ist, für eine Detektion spezifischer Signale in komplexen Signalverläufen nicht hinreichend differenziert.

Der Erfindung liegt deshalb die Aufgabe zugrunde, einen Signaldetektor der eingangs genannten Art zu schaffen, der eine automatische Einstellung der Detektionsempfindlichkeit bzw. -verstärkung ermöglicht, mit der Fehldetektionen sowohl bei schwankendem Pegel des spezifischem Nutzsignals als auch der unspezifischen Signalanteile vermieden werden.

Die Erfindung schließt die technische Lehre ein, bei einem Detektionsereignis die Amplitude des QRS-Komplexes zu ermitteln, um die Detektionsempfindlichkeit in Abhängigkeit davon zu herabzusetzen und anschließend die Detektionsempfindlichkeit wieder heraufzufahren, bis ein weiterer QRS-Komplex detektiert wird oder die Detektionsempfindlichkeit einen oberen Grenzwert erreicht, der von der Amplitude des vorangegangenen QRS-Komplexes abhängt. Die Detektionsempfindlichkeit kann hierbei zum einen durch eine Änderung der Eingangsverstärkung und zum anderen durch eine Änderung des Schwellwerts eingestellt werden.

Die Detektion eines Signalkomplexes - beispielsweise eines QRS-Komplexes - in dem Eingangssignal erfolgt bei dem erfindungsgemäßen Signaldetektor vorzugsweise - wie zuvor beschrieben - indem das Eingangssignal mittels einer Vergleichereinheit mit dem in einem ersten Speicherelement - im folgenden als Schwellwertspeicher bezeichnet - abgelegten Schwellwert verglichen wird. Überschreitet das Eingangssignal den Schwellwert, so wertet der Signaldetektor dies als Detektionsereignis und gibt ein entsprechendes Detektionssignal aus.

Der Begriff Eingangssignal ist hierbei und im folgenden allgemein zu verstehen und umfaßt unter anderem sämtliche elektrischen Signale, die eine beliebige biologische Zustandsgröße widerspiegeln. Vorzugsweise ist das Eingangssignal jedoch das über die Elektroden eines implantierten Herzschrittmachers abgenommene EKG-Signal, in dem die von spontanen Herzaktionen herrührenden QRS-Komplexe detektiert werden.

Die Begriffe Eingang und Ausgang bzw. Eingangssignal und Ausgangssignal bedeuten hierbei nicht, daß der erfindungsgemäße Signaldetektor notwendigerweise ein eigenständiges Gerät ist, das lediglich zum Datenaustausch mit anderen Geräten verbunden ist. Der erfindungsgemäße Signaldetektor eignet sich vielmehr auch zur Integration in andere Geräte, wie beispielsweise einen implantierbaren Herzschrittmacher.

In diesem Fall können Eingang und Ausgang Bestandteil einer rein softwaremäßig realisierten Datenschnittstelle sein.

In einer vorteilhaften Ausführungsform der Erfindung ist ein Sperrglied vorgesehen, das innerhalb eines vorgegebenen Zeitraums - Ausblend- oder Refraktärzeit ("holdoff time") - die Abgabe weiterer Detektionssignale sperrt und hierdurch die Mehrfachdetektion eines einzelnen Signalkomplexes verhindert.

Darüber hinaus - oder auch alternativ - erhöht der erfindungsgemäße Signaldetektor im Anschluß an ein Detektionsereignis den Schwellwert, um eine nachfolgende Fehldetektion beispielsweise einer T-Welle zu verhindern. Die Erhöhung des Schwellwerts wird hierbei an die Amplitude des zuvor detektierten Signalkomplexes gekoppelt, um den Schwellwert an das Signalniveau des Eingangssignals anzupassen. Die Schaltung weist deshalb Mittel auf, um den innerhalb eines vorgegebenen Zeitraums - im folgenden auch als Meßzeitraum bezeichnet - nach dem Erscheinen des Detektionssignals auftretenden Maximalwert des Eingangssignals zu bestimmen. Hieraus wird dann der erhöhte Schwellwert berechnet und - in einer zweckmäßigen Fortbildung - in einen Schwellwertspeicher geschrieben. In einer bevorzugten Ausführungsform wird der Schwellwert bei einem Detektionsereignis jeweils auf die Amplitude des detektierten Signalkomplexes angehoben, jedoch ist es auch möglich, den Schwellwert auf einen anderen Funktionswert der Amplitude zu erhöhen. Entscheidend ist lediglich, daß der Schwellwert an die Amplitude des zuvor detektierten Signalkomplexes angepaßt wird.

Der Meßzeitraum, innerhalb dessen der Maximalwert des Eingangssignals bestimmt wird, liegt hierbei bevorzugt innerhalb der Refraktärzeit und muß hinreichend groß sein, um den Abschnitt höchster Amplitude des Signalkomplexes zu umfassen. Bei der Detektion von QRS-Komplexen in einem EKG-Signal hat sich in Anbetracht der Maximaldauer einer R-Zacke ein Meßzeitraum von 70 bis 100 ms als geeignet erwiesen.

Der Maximalwert des detektierten Signalkomplexes dient jedoch nicht nur zur Berechnung des neuen, erhöhten Schwellwerts, sondern wird darüber hinaus dazu benutzt, einen variablen Grenzwert zu berechnen, bei dem ein anschließendes Herunterfahren des Schwellwerts beendet wird, um ein Absinken des Schwellwerts unter den Pegel von Störsignalen zu verhindern. Dazu wird er insbesondere zeitweilig in einem Amplitudenspeicher gespeichert.

Nach dem Meßzeitraum wird der Schwellwert in Richtung auf diesen Grenzwert abgesenkt. Hierzu weist die Schaltung insbesondere eine Recheneinheit auf, die jeweils aus dem aktuellen Schwellwert einen entsprechend verringerten Schwellwert für die nächste Absenkungsstufe berechnet und in den Schwellwertspeicher einschreibt.

Gemäß einer Variante der Erfindung erfolgt das Herunterfahren des Schwellwerts stufenweise gesteuert durch einen Taktgeber, indem die Recheneinheit bei jedem Impuls des Taktgebers aus dem aktuellen Schwellwert einen neuen Schwellwert berechnet und speichert. Bei der Detektion von QRS-Komplexen in einem EKG-Signal hat es sich als vorteilhaft erwiesen, den Schwellwert in Zeitabständen von jeweils 82 ms bei einer Messung im Atrium bzw. 125 ms bei einer Messung im Ventrikel herabzusetzen. Die Zeiträume, nach denen jeweils eine Verringerung des Schwellwerts erfolgt, müssen jedoch nicht notwendigerweise konstant sein. Vielmehr ist es bei einem stufenförmigen Herunterfahren des Schwellwerts oftmals vorteilhaft, die Breite der Stufen an die Amplitude des vorangegangenen Signalkomplexes anzupassen, um den Schwellwert nach einem "starken" Signalkomplex von dem relativ hohen Ausgangswert schnell wieder herunterfahren zu können, während der Schwellwert nach einem "schwachen" Signalkomplex nur langsam wieder heruntergefahren werden muß.

Die Berechnung des neuen Schwellwerts kann beispielsweise dadurch erfolgen, daß die Recheneinheit den aktuellen Schwellwert um einen vorgegebenen Betrag dekrementiert. Besonders vorteilhaft erfolgt die Berechnung jedoch in der Weise, daß der aktuelle Schwellwert zur Berechnung des neuen Schwellwerts jeweils mit einem vorgegebenen Faktor multipliziert wird. Auf diese Weise läßt sich ein schnelles Herunterfahren des Schwellwerts erreichen, was insbesondere dann wichtig ist, wenn vereinzelt Signalkomplexe mit besondere großer Amplitude auftreten, damit die Detektionsempfindlichkeit zur Detektion des folgenden Signalkomplexes mit normaler Amplitude ausreicht.

Das Absenken des Schwellwerts wird beim nächsten Detektionsereignis beendet, also wenn das Eingangssignal den Schwellwert übersteigt und der Schwellwertdiskriminator bzw. die Vergleichereinheit ein Detektionssignal erzeugt.

Um bei einem Ausbleiben des nächsten Detektionsereignisses ein übermäßiges Absinken des Schwellwerts zu verhindern, weist die Detektorschaltung mindestens einen Begrenzer auf. Der untere Grenzwert, bei dem das Herunterfahren des Schwellwerts von dem Begrenzer beendet wird, wird hierbei mittels einer Recheneinheit aus dem Maximalwert des zuvor detektierten Signalkomplexes berechnet, der im Amplitudenspeicher abgespeichert ist. Bei der Detektion von QRS-Komplexen in einem EKG-Signal hat es sich als vorteilhaft erwiesen, das Herunterfahren des Schwellwerts bei einem Pegel zu beenden, der 25% der Amplitude des zuvor detektierten QRS-Komplexes entspricht.

Der erfindungsgemäße Signaldetektor erhöht den Schwellwert also im Anschluß an ein Detektionsereignis und fährt den Schwellwert anschließend wieder herunter. Beim Herunterfahren des Schwellwerts wird hierbei sowohl der Startwert als auch der Endwert des Schwellwerts an die Amplitude des vorangegangenen QRS-Komplexes gekoppelt.

In einer vorteilhaften weiterbildenden Variante der Erfindung wird der Endwert beim Herunterfahren des Schwellwerts nicht nur an die Amplitude des letzten Signalkomplexes gekoppelt, sondern auch an die Amplituden vorangegangener Signalkomplexe. Hierzu weist der Signaldetektor mehrere Speicherelemente auf, die jeweils zur Speicherung der Amplitude eines Signalkomplexes dienen. In einer bevorzugten Ausführungsform dieser Variante wird dies mittels eines Schieberegisters mit mehreren Speicherplätzen realisiert. Bei der Detektion eines neuen Signalkomplexes wird dessen Amplitude dann als neuer Wert in das Schieberegister "hineingeschoben". Gleichzeitig wird der älteste Amplitudenwert aus dem Schieberegister herausgeschoben. Sämtliche Speicherelemente sind hierbei mit der zughörigen Recheneinheit verbunden, die den variablen unteren Grenzwert in Abhängigkeit von den gespeicherten Amplitudenwerten berechnet, wobei die Amplitudenwerte vorzugsweise entsprechend der Reihenfolge ihrer vorausgegangen Bestimmung gewichtet werden. So ist beispielsweise sinnvoll, die Amplituden der unmittelbar vorausgegangenen Signalkomplexe stärker zu gewichten als die Amplituden älterer Signalkomplexe.

Bei der letztgenannten Variante wird der Einfluß eines "Ausreißers" vorteilhaft verringert. Auch erfolgt die Anpassung des Schwellwerts an das Signalniveau des Eingangssignals genauer, da mehrere Amplitudenwerte das tatsächliche Signalniveau besser widerspiegeln als ein einzelner Amplitudenwert. In der gleichen Weise läßt sich auch der zu Beginn des Herunterfahrens wirksame Initialwert der Detektionsschwelle an die Amplituden mehrerer aufeinanderfolgender Signalkomplexe anpassen.

In einer bevorzugten Ausführungsform ist zusätzlich zu dem erwähnten Begrenzer ein weiterer Begrenzer vorgesehen, der unabhängig von der Amplitude der vorangegangenen Signalkomplexes ein Absinken des Schwellwerts unter einen vorgegebenen (absoluten) unteren Grenzwert verhindert.

Der vorstehend beschriebene Signaldetektor eignet sich vorteilhaft zur Realisierung im Rahmen einer softwaremäßigen Lösung, insbesondere als Bestandteil eines mikroprozessorgesteuerten Herzschrittmachers oder Defibrillators. Er ist jedoch nicht auf eine primär softwaremäßige Realisierung beschränkt, sondern läßt sich auch rein hardwaremäßig oder mit einer Kombination von Hardware-Komponenten und einer durch Software realisierten Steuerung ausführen.

Wichtig ist weiterhin, daß der erfindungsgemäße Signaldetektor nicht auf eine separate Ausführung der vorstehend beschriebenen Bauelemente bzw. Baugruppen beschränkt ist. So können die einzelnen Recheneinheiten beispielsweise Bestandteil einer arithemtischen Einheit (engl. ALU - arithmetical logical unit) sein. Auch können die verschiedenen Steuereinheiten bzw. Regelschaltungen in einer Baugruppe zusammengefaßt sein. Maßgebend ist lediglich, daß die vorstehend beschriebenen technischen Funktionen zur Verfügung gestellt werden.

Andere vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
- Figur 1: als bevorzugtes Ausführungsbeispiel der Erfindung einen Signaldetektor zur Detektion von QRS-Komplexen in einem Elektrokardiogramm in einer Darstellung als Blockschaltbild,
- Figur 2: die Eingangsstufe des Signaldetektors aus Figur 1 als Blockschaltbild sowie
- die Figuren 3a bis 3d: jeweils exemplarisch ein Elektrokardiogramm sowie den zugehörigen zeitlichen Verlauf der Detektionsschwelle bei verschiedenen Ausführungsformen der Erfindung.

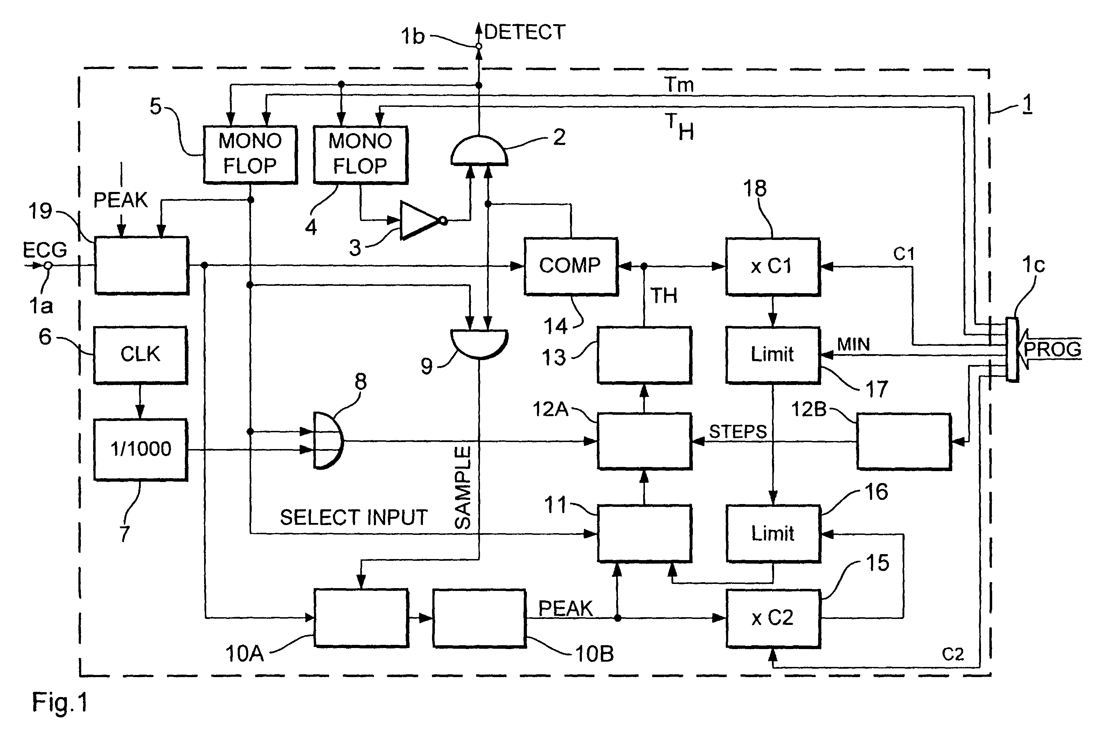

Der in Figur 1 dargestellte Signaldetektor 1 ist Bestandteil eines implantierbaren Herzschrittmachers und dient zur Detektion von über einen Detektoreingang 1a zugeführten QRS-Komplexen in einem über die Schrittmacherelektroden intrakardial abgenommenen Elektrokardiogramm ECG. Die Detektion der QRS-Komplexe und die Ausgabe eines Detektionssignals über einen Ausgang 1b erfolgt hierbei zum einen, um im Demand-Betrieb beim Auftreten spontaner Herzaktionen die Abgabe von Stimulationsimpulsen inhibieren zu können. Zum anderen ermöglicht sie die Bestimmung der Herzrate und damit die Erkennung von Arrhythmien, insbesondere Tachykardien, und die Einleitung eines geeigneten Therapiemodus.

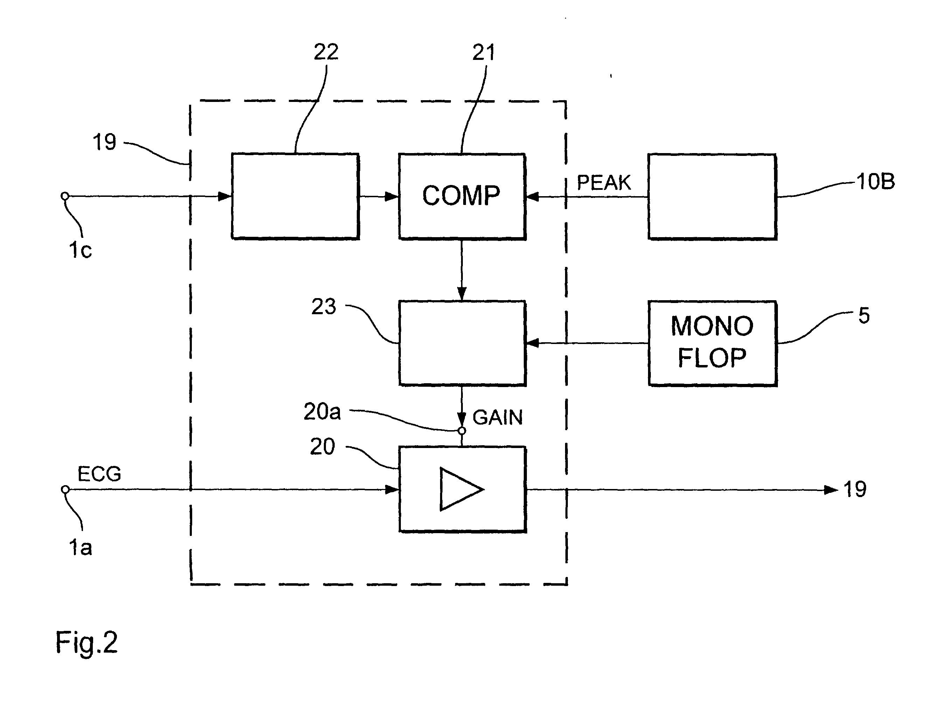

Zur Aufnahme des EKG-Signals enthält der Signaldetektor 1 eine mit dem Eingang 1a verbundene Eingangsstufe 19, die genauer in Figur 2 dargestellt ist und das EKG-Signal verstärkt, um die anschließende Detektion zu vereinfachen. Das Ausgangssignal der Eingangsstufe 19 wird einer Vergleichereinheit (einem Schwellwertdiskriminator) 14 zugeführt und dort mit einem Schwellwert verglichen, der in einem Schwellwertspeicher 13 abgespeichert ist. Liegt die Amplitude des EKG-Signals unter dem Schwellwert, so nimmt das Ausgangssignal des Schwellwertdiskriminators 14 Low-Pegel an, der über ein UND-Gatter 2 an den Ausgang 1b des Signaldetektors 1 weitergegeben wird. In diesem Fall wird, wie weiter unten genauer beschrieben ist, die Detektionsempfindlichkeit stufenweise erhöht, um die Detektion von QRS-Komplexen mit einer geringen Amplitude zu ermöglichen.

Hierzu wird zunächst der im Schwellwertspeicher 13 abgelegte aktuelle Schwellwert einer Recheneinheit 18 zugeführt, die durch Multiplikation mit einem vorgegebenen Faktor C1 einen entsprechend verringerten Schwellwert berechnet. Das Ausgangssignal der Recheneneinheit 18 - der verringerte Schwellwert - wird einem ersten Begrenzer 17 zugeführt, der den Schwellwert nach unten auf einen durch Programmierung (über einen Programmieranschluß 1c) vorgegebenen Grenzwert MIN begrenzt. Unabhängig von dem durch die Recheneinheit 18 berechneten verringerten Schwellwert ist das Ausgangssignal des Begrenzers 17 also mindestens gleich dem unteren Grenzwert MIN, was eine Fehldetektion von Störsignalen wirksam verhindert.

Das Ausgangssignal des ersten Begrenzers 17 wird einem zweiten Begrenzer 16 zugeführt, der den Schwellwert nochmals nach unten begrenzt. Der im Begrenzer 16 eingestellte Minimalwert ist jedoch nicht konstant, sondern abhängig von der Amplitude des letzten QRS-Komplexes, die bei jeder Detektion durch einen Spannungsmesser 10A neu bestimmt und in einem Amplitudenspeicher 10B abgespeichert wird. Die Amplitude des letzten QRS-Komplexes wird aus dem Amplitudenspeicher 10B ausgelesen und einer zweiten Recheneinheit 15 zugeführt, die durch Multiplikation mit einem Faktor C2 den aktuellen Minimalwert für den Begrenzer 16 berechnet. Beträgt der Wert von C2 beispielsweise 0,25, so verhindert der zweite Begrenzer 16 ein Absinken des Schwellwerts unter ein Viertel der Amplitude des letzten Detektionsereignisses.

Der auf diese Weise limitierte Schwellwert wird dann einer Auswahleinheit 11 zugeführt und von dieser zu der Steuereinheit 12 weitergeleitet, die die Aufgabe hat, eine stufenweise Verringerung des Schwellwerts zeitlich zu steuern. Hierzu weist der Signaldetektor einen Taktgeber 6 auf, dessen hochratiges Taktsignal über einen Frequenzteiler 7 und das ODER-Gatter 8 einer Zeitsteuereinheit 12A zugeführt wird. Diese ist zudem mit einem über den Programmiereingang 1c programmierbaren Zeitstufenspeicher 12B verbunden und schreibt unter Taktung durch den internen Taktgeber und gemäß einem im Zeitstufenspeicher 12B gespeicherten Zeitablauf für die Einstellung des Schwellwertes den an ihrem Eingang anliegenden verringerten Schwellwert in den Schwellwertspeicher 13. Unmittelbar nach diesem Schreibvorgang ist die Detektionsempfindlichkeit des Signaldetektors entsprechend dem neuen Schwellwert eingestellt. Gleichzeitig wird in der vorstehend beschriebenen Weise ein neuer Schwellwert berechnet und nach Ablauf der im gespeicherten Zeitablauf für die neue Stufe vorgesehenen Zeitspanne wiederum in den Schwellwertspeicher 13 eingeschrieben. Der Schwellwert wird also stufenweise jeweils um den Faktor C1 verringert, bis entweder der erste Begrenzer 17 ein Absinken des Schwellwerts unter den absoluten Minimalwert verhindert oder der zweite Begrenzer 16 einsetzt, um ein Absinken des Schwellwerts unter das C2-fache der Amplitude des letzten QRS-Komplexes zu verhindern.

Neben den beiden vorstehend genannten Ausstiegsbedingungen wird das programmierte Absenken des Schwellwerts auch beendet, wenn ein QRS-Komplex detektiert wird. Dies ist der Fall, wenn das von der Eingangsstufe 19 verstärkte EKG-Signal den im Schwellwertspeicher 13 abgespeicherten aktuellen Wert übersteigt, so daß das Ausgangssignal der Vergleichereinheit 14 High-Pegel annimmt. Der am Ausgang der Vergleichereinheit 14 erscheinende High-Pegel wird über das UND-Gatter 2 zum einen dem Ausgang 1b zugeführt und dort als Detektionssignal DETECT ausgegeben. Zum anderen triggert es bei der Detektion eines QRS-Komplexes ein erstes Monoflop 4, das daraufhin für eine - ebenfalls programmierbare - Zeitspanne T_{H} (z.B. 121 ms) Low-Pegel annimmt, der über einen Inverter 3 dem UND-Gatter 2 zugeführt wird und dieses sperrt, so daß während der Haltezeit des Monoflops 4 unabhängig vom Ausgangssignal der Vergleichereinheit 14 keine weiteren Detektionssignale abgegeben werden können.

Darüber hinaus triggert das Ausgangssignal der Vergleichereinheit 14 ein zweites Monoflop 5, das anschließend für eine - wiederum programmierte - Zeitspanne Tₘ (z.B. 86 ms) High-Pegel annimmt. Das Monoflop 5 definiert das Zeitfenster nach einem Detektionsereignis, in dem der als Spitzenwertdetektor ausgebildete Spannungsmesser 10A aktiviert ist und die Amplitude des detektierten QRS-Komplexes ermittelt.

Die Vergleichereinheit 14 vergleicht hierzu laufend die Amplitude des EKG-Signals mit dem im Schwellwertspeicher 13 abgelegten Schwellwert. Beim Überschreiten des Schwellwerts nimmt das Ausgangssignal der Vergleichereinheit High-Pegel an, der den beiden UND-Gattern 2, 9 zugeführt wird. Während das UND-Gatter 2 nun wegen des zuvor getriggerten MONO-FLOPs sperrt, schaltet das andere UND-Gatter 9 durch und leitet das Ausgangssignal der Vergleichereinheit an den Spannungsmesser 10A weiter, der ausgangsseitig mit dem Amplitudenspeicher 10B verbunden ist und die innerhalb des Zeitfensters erfaßte Maximalamplitude dort als aktuellen Maximalwert abspeichert. Während der Haltezeit des Monoflops 5 liest die Auswahleinheit - anders als bei dem zuvor beschriebenen Absenken des Schwellwerts - den Maximalwert PEAK aus dem Amplitudenspeicher 10B aus und führt diesen der Zeitsteuereinheit 12A zu, die ebenfalls über das Monoflop 5 und das ODER-Gatter 8 angesteuert wird und den Maximalwert als neuen Schwellwert TH in den Schwellwertspeicher 13 einschreibt. (Falls das EKG-Signal während der Haltezeit Tₘ des Monoflops 5 den im Speicher 13 gespeicherten Schwellwert nochmals überschreitet, so werden der Maximalwert im Speicher 10B und der - beim vorliegenden Beispiel identische - Schwellwert im Speicher 13 entsprechend aktualisiert. Der Maximal- und Schwellwert wird also während der Detektion eines QRS-Komplexes nachgeführt, bis die Haltezeit Tₘ des Monoflops 5 abgelaufen ist.)

Der in dem Amplitudenspeicher 10B abgelegte Maximalwert PE-AK dient hierbei - wie bereits beschrieben - zur Berechnung des unteren Grenzwerts für den Begrenzer 16 beim anschließenden Herunterfahren des Schwellwerts zur Erhöhung der Detektionsempfindlichkeit und wird deshalb nach Ablauf der Haltezeit des Monoflops 5 nicht mehr verändert. Dies wird dadurch erreicht, daß das Ausgangssignal der Vergleichereinheit 14 nach Ablauf der Haltezeit des Monoflops 5 von dem UND-Gatter 9 gesperrt wird, so daß der Speicher 10B unabhängig vom Ausgangssignal der Vergleichereinheit 14 seinen Wert beibehält.

Im Gegensatz dazu wird der im Schwellwertspeicher 13 abgelegte Schwellwert TH - wie bereits vorstehend beschrieben-zur Erhöhung der Detektionsempfindlichkeit stufenweise heruntergefahren.

Wesentliche Elemente der Eingangsstufe 19 sind in Figur 2 dargestellt.

Zur Verstärkung des intrakardial abgenommenen EKG-Signals ist ein Verstärker 20 vorgesehen, dessen Verstärkungsfaktor einstellbar ist, um eine Anpassung an den Signalpegel des EKG-Signals ECG zu ermöglichen. Die Einstellung des Verstärkungsfaktors erfolgt deshalb in Abhängigkeit von den Amplituden der letzten QRS-Komplexe, die jeweils in dem Amplitudenspeicher 10B abgespeichert sind. Der abgelegte Maximalwert wird deshalb einer Vergleichereinheit 21 zugeführt und von dieser mit einem in einem Speicherelement 22 abgelegten Schwellwert verglichen. Überschreitet der Maximalwert des letzten QRS-Komplexes den vorgegebenen Schwellwert, so nimmt das Ausgangssignal der Vergleichereinheit 21 High-Pegel an, der einer Eingangsstufen-Steuereinheit 23 zugeführt wird, die die Aufgabe hat, den Verstärkungsfaktor des Eingangsverstärkers 20 in Abhängigkeit von den Amplituden der letzten QRS-Komplexes einzustellen. Eine Änderung des Verstärkungsfaktors erfolgt hierbei - falls überhaupt erforderlich - jeweils dann, wenn der Maximalwert des aktuellen QRS-Komplexes bestimmt worden ist. Dies ist der Fall, wenn die Haltezeit des Monoflops 5 abgelaufen ist und dieses wieder Low-Pegel annimmt. Das Monoflop 5 ist deshalb mit der Steuereinheit 23 verbunden und triggert diese mit der fallenden Flanke des Ausgangssignals, also am Ende der Haltezeit. Die Steuereinheit 23 überprüft daraufhin das Ausgangssignal der Vergleichereinheit 21.

Liefert die Vergleichereinheit 21 High-Pegel, so bedeutet dies, daß der Maximalwert des aktuellen QRS-Komplexes den vorgegebenen Schwellwert überschreitet. In diesem Fall stellt die Steuereinheit 23 einen verringerten Verstärkungsfaktor ein, da der Pegel des EKG-Signals relativ hoch ist. Darüber hinaus speichert die Steuereinheit 23 bei jeder Triggerung durch die fallende Flanke des Monoflops 5 das Ausgangssignal der Vergleichereinheit 21 (was hier nicht näher gezeigt ist) intern ab, um bei der Entscheidung über die Änderung des Verstärkungsfaktors auch die Maximalwerte der vorangegangenen QRS-Komplexe berücksichtigen zu können.

Erhält die Steuereinheit 23 dagegen einen Low-Pegel von der Vergleichereinheit 21, so bedeutet dies, daß der aktuelle QRS-Komplex einen relativ geringen Pegel aufweist. In diesem Fall zieht die Steuereinheit 23 zur Entscheidung über eine Änderung des Verstärkungsfaktors die intern abgespeicherten Ausgangssignale der beiden vorangegangenen QRS-Komplexe heran. Die aktuelle Einstellung des Verstärkungsfaktors wird beibehalten, wenn der Maximalwert mindestens eines der beiden vorangegangenen QRS-Komplexe den vorgegebenen Schwellwert überschritten hat. Falls jedoch nicht nur der Maximalwert des aktuellen QRS-Komplexes den vorgegebenen Schwellwert unterschreitet, sondern auch die Maximalwerte der beiden vorangegangenen QRS-Komplexe, so stellt die Steuereinheit 23 einen erhöhten Verstärkungsfaktor ein, um eine Anpassung an das relativ geringe Signalniveau des EKG-Signals zu ermöglichen.

Der Verstärkungsfaktor des Eingangsverstärkers 20 wird also verringert, sobald ein einzelner QRS-Komplex mit hoher Amplitude detektiert wird, während die Verringerung des Verstärkungsfaktors erst erfolgt, wenn nacheinander drei QRS-Komplexe mit niedriger Amplitude detektiert werden.

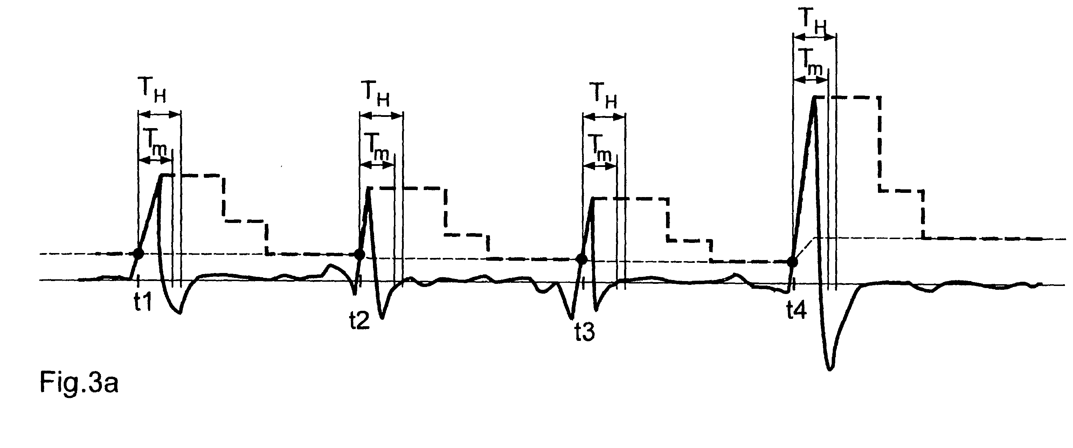

Die Figuren 3a bis 3d zeigen jeweils den Verlauf einen typischen EKG-Signals sowie den Schwellwert eines Signaldetektors gemäß verschiedenen Ausführungsformen der Erfindung. Das EKG-Signal ist jeweils als durchgezogen Linie dargestellt und umfaßt drei QRS-Komplexe mit relativ geringer Amplitude und anschließend einen bzw. zwei QRS-Komplexe mit etwas erhöhter Amplitude. Der zeitliche Verlauf des Schwellwertes des Signaldetektors ist als gestrichelte dikke Linie dargestellt. Weiterhin zeigen die Figuren jeweils den variablen unteren Grenzwert des Schwellwerts als dünne gestrichelte Linie.

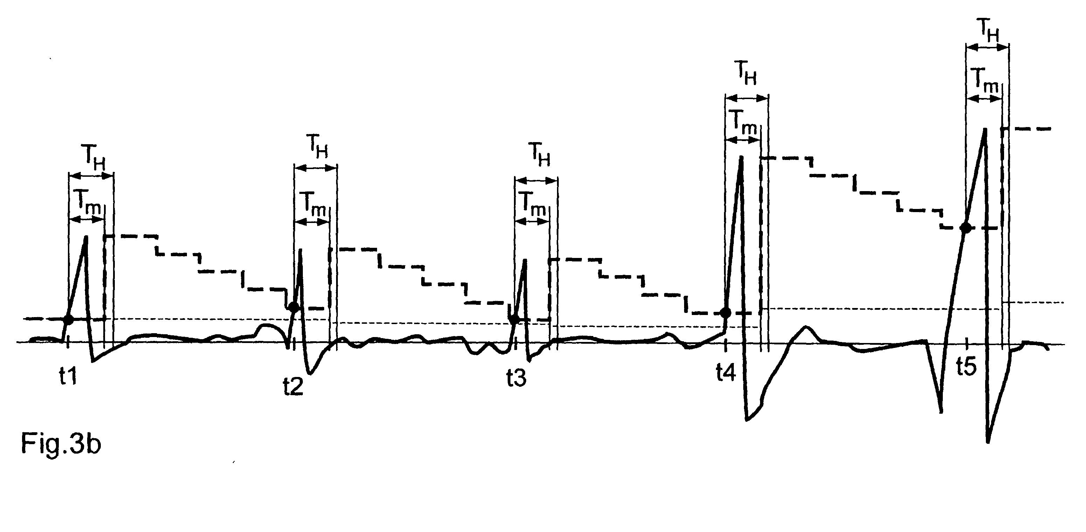

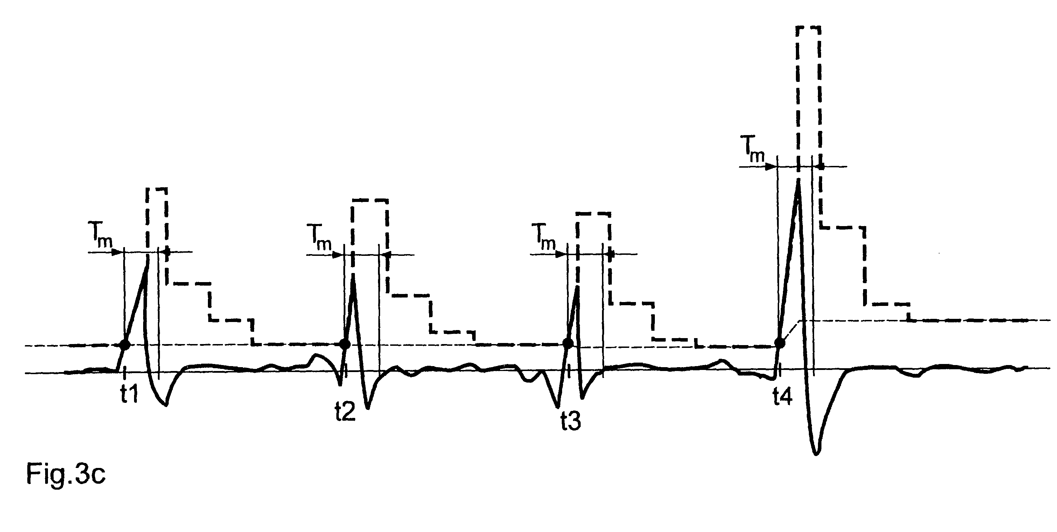

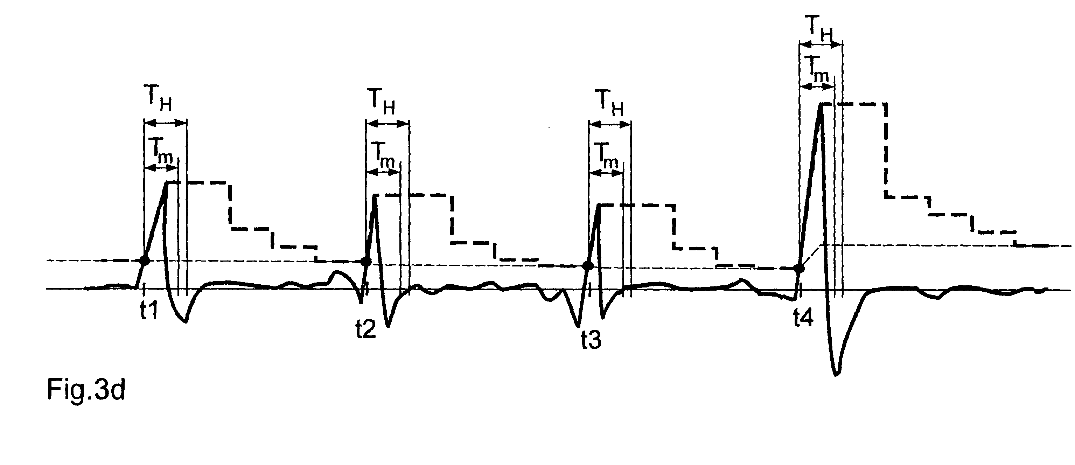

In allen Figuren wird von dem aktuellen unteren Grenzwert ausgegangen. Zum Zeitpunkt t₁ überschreitet das EKG-Signal den Schwellwert, was zu einem Detektionsereignis führt (als schwarzer Punkt dargestellt). Im Anschluß daran startet bei Fig. 3a, 3b und 3d eine Ausblend- oder Refraktärzeit T_{H} (holdoff time), innerhalb derer keine weiteren Detektionssignale abgegeben werden, um zu verhindern, daß ein QRS-Komplex mehrfach detektiert wird. Bei Fig. 3c ist keine Refraktärzeit vorgesehen.

Zugleich startet jedes Detektionsereignis zu den Zeitpunkten t₁ bis t₄ bzw. t₅ eine zweite Zeitspanne (Meßzeitfenster) Tₘ, innerhalb derer die maximale Signalamplitude bestimmt wird. Der Schwellwert wird gemäß Fig. 3a, 3c und 3d dem EKG-Signal bis zum Erreichen des Maximalwerts nachgeführt, während gemäß Fig 3b die Neueinstellung der Nachweisschwelle erst nach Schließung des Meßzeitfensters erfolgt. Ein Detektionsereignis führt zunächst zu einer annähernd sprungartigen Verringerung der Detektionsempfindlichkeit. Gemäß Fig. 3a, 3b und 3d wird dabei die Nachweisschwelle bis auf die Maximalamplitude der zuletzt erfaßten R-Welle erhöht, während gemäß Fig. 3c sogar eine stufenartige kurzzeitige Erhöhung deutlich darüber hinaus vorgesehen ist, die die Ausblendung von Artefakten auch unter Verzicht auf eine übliche absolute Ausblend- oder Refraktärzeit ermöglicht.

Im Anschluß daran wird die Detektionsempfindlichkeit stufenweise wieder erhöht, der Schwellwert also entsprechend stufenweise verringert. Dies geschieht gemäß Fig. 3a - entsprechend dem oben unter Bezugnahme auf Fig. 1 beschriebenen Prinzip - jeweils durch Halbierung des vorhergehenden Wertes in jeder neuen Stufe, gemäß Fig. 3c (nach Zurückführung der initialen Schwellwert-Überhöhung) ebenso, gemäß Fig. 3b in betragsmäßig konstanten Stufen, und gemäß Fig. 3d in einer Kombination aus den in Fig. 3a und 3c gezeigten Vorgehensweisen.

Ausgangspunkt ist bei allen Beispielen der maximale Amplitudenwert des letzten QRS-Komplexes, dieser kann aber zur Festlegung des Initialwertes auch mit einem vorbestimmten Faktor multipliziert oder ggfs. mit einem festen Inkrement oder Dekrement versehen sein - was bei der Anordnung nach Fig. 1 über eine entsprechende Ausführung der Auswahleinheit 11 oder der Zeitsteuereinheit 12 zu realisieren ist.

In allen Figuren weisen die einzelnen Stufen die gleiche Breite (Zeitdauer) auf, für die bei der QRS-Erfassung beispielsweise 125 ms oder in einem atrialen Nachweiskanal 82 ms eingestellt sein können. Es kann jedoch auch eine Zeitsteuerung der Nachweisschwelle mit unterschiedlich breiten Stufen programmiert sein, oder die Stufenbreite bzw. -dauer kann in Abhängigkeit von der Signalamplitude festgelegt, insbesondere bei schwachen Signalen vergrößert werden

Das Absenken des Schwellwertes wird beendet, wenn dieser den variablen unteren Grenzwert - als dünne gestrichelte Linie dargestellt - erreicht. Der fest programmierte absolute untere Grenzwert ist in den Figuren nicht dargestellt.

Neben den beiden vorstehend genannten Ausstiegsbedingungen wird - wie die Figuren zeigen - das Herunterfahren des Schwellwerts auch beendet, wenn der Signaldetektor den nächsten QRS-Komplex detektiert. In diesem Fall wird die Detektionsempfindlichkeit wieder - wie vorstehend beschrieben - nahezu sprungartig erhöht.

Die Erfindung beschränkt sich in ihrer Ausführung nicht auf die vorstehend angegebenen bevorzugten Ausführungsbeispiele. Vielmehr ist eine Vielzahl von Varianten denkbar, welche von der dargestellten Lösung auch bei grundsätzlich anders gearteten Ausführungen Gebrauch machen. Abwandlungen können beispielsweise für die spezifische Erfassung von P-Wellen in einem komplexen EKG dahingehend zweckmäßig sein, daß nach einem vorgegebenen Zeitprogramm zunächst eine längerwährende Erhöhung der Nachweisschwelle über die Amplitude der P-Welle hinaus zur Ausblendung von störenden Kammerimpulsen - die etwa als Fernfeldsignale im Vorhof auftreten können - und erst später eine Absenkung zur Erfassung der nächstfolgenden P-Welle realisiert wird.

Weiterhin kann eine differenzierte Handhabung der oben skizzierten Funktionsweise eines QRS-Detektors bei spontanen Herzaktionen einerseits und nach Stimulationsimpulsen andererseits zweckmäßig sein.

Es kann etwa eine Verkürzung der Zeitdauer der Stufen nach einem Stimulus - gegenüber spontanen Herzaktionen - um einen vorbestimmten Faktor (z.B. 2) vorgesehen sein, die bei einer Anordnung nach Fig. 1 beispielsweise über eine zusätzliche Signalverbindung der Zeitsteuerung 12A zum Impulsgenerator eines Schrittmacherteils realisiert wird. Weiterhin oder zusätzlich kann auf einen Stimulus hin ein Detektionsparameter - insbesondere die Verstärkung der Eingangsstufe - auf einen anderen Wert eingestellt werden als nach einer spontanen Herzaktion. Hierzu kann bei der Anordnung nach Fig. 2 zusätzlich eine Steuerverbindung von einem Schrittmacher-Impulsgenerator zur Eingangsstufen-Steuereinheit 23 vorgesehen sein.

## Patentansprüche

1. Signaldetektor (1) zur Detektion eines Biosignals mit annähernd bekannter Morphologie in einem gestörten Eingangssignal (ECG), insbesondere zur Detektion von QRS-Komplexen in einem Elektrokardiogramm, mit
einem Detektoreingang zur Aufnahme des Eingangssignals (ECG) und einem Detektorausgang zur Abgabe eines die Detektion des Biosignals in dem Eingangssignal anzeigenden Detektionssignals (DETECT),
einem signalseitig mit dem Detektoreingang verbundenen Schwellwertdiskriminator (14) zum Vergleich des Eingangs-signals (ECG) mit einem Detektions-Schwellwert und zur Ausgabe des Detektionssignals (DETECT) bei Überschreitung des Schwellwerts durch das Eingangssignal,
einer über einen Signaleingang mit dem Detektoreingang (1a) und über einen Steuereingang mit dem Ausgang des Schwellwertdiskriminators (14) verbundenen Einrichtung (10A) zur Erfassung der maximalen Amplitude des detektierten Signalkomplexes innerhalb eines vorgegebenen Zeitfensters (Tₘ) nach Ausgabe des Detektionssignals (DETECT),
einer über einen Signaleingang mit dem Amplitudenerfassungseinrichtung (10A) und über einen Steuereingang mit dem Schwellwertdiskriminator (14) und ausgangsseitig mindestens mittelbar mit dem Steuereingang des Schwellwertdiskriminators verbundenen Detektorparameter-Vorwahlschaltung (11) zur Bestimmung eines Initialwertes eines die Detektionsempfindlichkeit kennzeichnenden Detektorparameters, der den Detektions-Schwellwert bestimmt, im Ansprechen auf das Detektionssignal und in Abhängigkeit von der maximalen Amplitude (PEAK) und
einer ausgangsseitig mit dem Steuereingang des Schwellwertdiskriminators (14) verbundenen Detektorparameter-Zeitsteuerschaltung (11, 12A) zur Einstellung einer vorbestimmten Zeitabhängigkeit des Detektorparameters und damit des Detektions-Schwellwertes (TH), ausgehend von dem Initialwert,
wobei die vorbestimmte Zeitabhängigkeit mindestens in einem ersten Zeitbereich eine stufenartige Erhöhung bis höchstens zu einem in Abhängigkeit von der maximalen Amplitude bestimmten oberen Grenzwert und in einem zweiten Zeitbereich eine stufenartige Verringerung des Detektions-Schwellwertes bis höchstens zu einem unteren Grenzwert mit vorbestimmten Stufen (STEPS) einschließt, **dadurch gekennzeichnet, daß** der untere Grenzwert in Abhängigkeit von der maximalen Amplitude (PEAK) bestimmt ist.

2. Signaldetektor nach Anspruch 1, **dadurch gekennzeichnet, daß** ein mit der Amplitudenerfassungseinrichtung (10A) verbundener Amplitudenspeicher (10B) zur Speicherung der maximalen Amplitude des Eingangssignals in dem vorbestimmten Zeitfenster und eine mit dem Amplitudenspeicher verbundene Recheneinheit (15) zur Berechnung des unteren und/oder oberen Grenzwerts für den Detektions-Schwellwert in Abhängigkeit von der gespeicherten Amplitude sowie ein eingangsseitig mit dem Ausgang der Recheneinheit (15) verbundener erster Begrenzer (16) für den Detektions-Schwellwert (TH) vorgesehen sind.

3. Signaldetektor nach Anspruch 2, **dadurch gekennzeichnet**, die ein mit dem ersten Begrenzer (16) in Reihe geschalteter zweiten Begrenzer (17) zur Begrenzung des Detektions-Schwellwertes (TH) auf einen fest vorgegebenen absoluten Grenzwert vorgesehen ist.

4. Signaldetektor nach einem der vorhergehenden Ansprüche, **dadurch gekennzeichnet, daß** dem Ausgang ein Sperrglied (2, 3, 4) vorgeschaltet ist zur Sperrung der Abgabe eines weiteren Detektionssignals (DETECT) innerhalb eines vorbestimmten Zeitraums (T_{H}) nach einem Detektionssignal.

5. Signaldetektor nach einem der vorhergehenden Ansprüche, **dadurch gekennzeichnet, daß** der Detektorparameter-Steuerschaltung (11, 12A) ein Zeitstufenspeicher (12B) zur, insbesondere programmierbaren, Speicherung mindestens einer, insbesondere in Abhängigkeit von einer bekannten Signalmorphologie vorbestimmten, Zeitabhängigkeit des Detektions-Schwellwertes zugeordnet ist.

6. Signaldetektor nach einem der vorhergehenden Ansprüche, **dadurch gekennzeichnet, daß** ein Schwellwertspeicher (10B) und eine mit dem Schwellwertspeicher verbundene Recheneinheit (18) zur Änderung des Empfindlichkeitsparameters und somit des Detektions-Schwellwerts um einen vorgegebenen, insbesondere programmierbaren, Faktor oder ein vorgegebenes Inkrement oder Dekrement vorgesehen sind.

7. Signaldetektor nach einem der vorhergehenden Ansprüche, **gekennzeichnet durch**
einen Eingangsverstärker (20) mit einem Steuereingang (20a) zur Einstellung eines Verstärkungsfaktors,
einen Amplituden-Klassifizierungsspeicher (22) zur Speicherung eines Klassifizierungs-Schwellwerts zur Unterscheidung starker und schwacher Detektionsereignisse und
eine eingangsseitig mit dem Amplituden-Klassifizierungsspeicher (22) und dem Detektoreingang (1a) und ausgangsseitig mindestens mittelbar mit dem Eingangsverstärker-Steuereingang (20a) verbundene Klassifizierungs-Vergleichereinheit (21) zum Vergleich des Eingangssignals mit dem gespeicherten Klassifizierungs-Schwellwert und zur Erzeugung eines Steuersignals an den Eingangsverstärker beim Überschreiten des Schwellwerts.

## Claims

1. A signal detector (1) for the detection of a biosignal of approximately known morphology in a disturbed input signal (ECG), in particular for the detection of QRS-complexes in an electrocardiogram, comprising
a detector input for receiving the input signal (ECG) and a detector output for delivering a detection signal (DETECT) indicating detection of the biosignal in the input signal,
a threshold value discriminator (14) connected on the signal side to the detector input for comparison of the input signal (ECG) to a detection threshold value and for output of the detection signal (DETECT) when the threshold value is exceeded by the input signal,
a device (10A) connected by way of a signal input to the detector input (1a) and by way of a control input to the output of the threshold value discriminator (14) for detecting the maximum amplitude of the detected signal complex within a predetermined time window (Tₘ) after output of the detection signal (DETECT),
a detector parameter preselection circuit (11) connected by way of a signal input to the amplitude detection device (10A) and by way of a control input to the threshold value discriminator (10) and at the output side at least indirectly to the control input of the threshold value discriminator, for determining an initial value of a detector parameter which characterises the level of detection sensitivity and which determines the detection threshold value, in response to the detection signal and in dependence on the maximum amplitude (PEAK), and
a detector parameter time control circuit (11, 12A) connected at the output side to the control input of the threshold value discriminator (14), for setting a predetermined time dependency of the detector parameter and therewith the detection threshold value (TH), starting from the initial value,
wherein the predetermined time dependency includes at least in a first time range a stepped increase to at most an upper limit value determined in dependence on the maximum amplitude and in a second time range a stepped reduction in the detection threshold value to at most a lower limit value in predetermined steps (STEPS), **characterised in that** the lower limit value is determined in dependence on the maximum amplitude (PEAK).

2. A signal detector according to claim 1 **characterised in that** there are provided an amplitude memory (10B) connected to the amplitude detection device (10A) for storage of the maximum amplitude of the input signal in the predetermined time window and a computing unit (15) connected to the amplitude memory for computation of the lower and/or upper limit value for the detection threshold value in dependence on the stored amplitude and a first limiter (16) connected on the input side to the output of the computing unit (15) for the detection threshold value (TH).

3. A signal detector according to claim 2 **characterised in that** there is provided a second limiter (17) connected in series with the first limiter (16) for limiting the detection threshold value (TH) to a fixedly predetermined absolute limit value.

4. A signal detector according to one of the preceding claims **characterised in that** connected upstream of the output is a blocking member (2, 3, 4) for blocking the delivery of a further detection signal (DETECT) within a predetermined period of time (T_{H}) after a detection signal.

5. A signal detector according to one of the preceding claims **characterised in that** associated with the detector parameter control circuit (11, 12A) is a time step memory (12B) for in particular programmable storage of at least one time dependency, which is predetermined in particular in dependence on a known signal morphology, of the detection threshold value.

6. A signal detector according to one of the preceding claims **characterised in that** there are provided a threshold value memory (10B) and a computing unit (18) connected to the threshold value memory for alteration of the sensitivity parameter and thus the detection threshold value by a predetermined and in particular programmable factor or a predetermined increment or decrement.

7. A signal detector according to one of the preceding claims **characterised by**
an input amplifier (20) having a control input (20a) for adjusting the gain factor,
an amplitude classification memory (22) for storage of a classification threshold value for distinguishing strong and weak detection events, and
a classification comparator unit (21) connected at the input side to the amplitude classification memory (22) and the detector input (1a) and on the output side at least indirectly to the input amplifier control input (20a) for comparison of the input signal to the stored classification threshold value and for producing a control signal to the input amplifier when the threshold value is exceeded.

## Revendications

1. Détecteur de signaux (1) pour détecter un signal bio possédant une morphologie approximativement connue dans un signal d'entrée perturbé (ECG), notamment pour la détection de complexes QRS dans un électrocardiogramme, comportant
une entrée du détecteur pour recevoir le signal d'entrée (ECG) et une sortie du détecteur pour délivrer un signal de détection (DETECT) indiquant la détection du signal bio dans le signal d'entrée,
un discriminateur à valeur de seuil (14), qui est relié côté signal à l'entrée du détecteur et sert à comparer le signal d'entrée (ECG) à une valeur de seuil de détection et à délivrer le signal de détection (DETECT) lors du dépassement de la valeur de seuil par le signal d'entrée,
un dispositif (10A) qui est relié par l'intermédiaire d'une entrée de signaux à l'entrée (1a) du détecteur et par l'intermédiaire d'une entrée de commande à la sortie du discriminateur à valeur de seuil (14) et sert à détecter l'amplitude maximale du complexe de signal détecté à l'intérieur d'une fenêtre temporelle prédéterminée (Tₘ) après la délivrance du signal de détection (DETECT),
un circuit (11) de présélection de paramètres du détecteur qui est relié par l'intermédiaire d'une entrée du signal au dispositif de détection d'amplitude (10A) et par l'intermédiaire d'une entrée de commande au discriminateur à valeur de seuil (14) et, côté sortie, au moins indirectement à l'entrée de commande du discriminateur à valeur de seuil, qui sert à déterminer une valeur initiale d'un paramètre du détecteur, qui caractérise la sensibilité de détection et détermine la valeur de seuil de détection, en réponse au signal de détection et en fonction de l'amplitude maximale (PEAK), et
un circuit (11, 12A) de commande temporelle des paramètres de détection, qui est relié côté sortie à l'appareil de commande du discriminateur à valeur de seuil (14) et sert à régler une dépendance prédéterminée, vis-à-vis du temps, du paramètre du détecteur et par conséquent de la valeur de seuil de détection (TH), à partir de la valeur initiale,
dans lequel la dépendance temporelle prédéterminée inclut au moins dans une première plage temporelle un accroissement échelonné jusqu'à, au maximum, une valeur limite supérieure déterminée en fonction de l'amplitude maximale, et dans une seconde plage temporelle une réduction échelonnée de la valeur de seuil de détection jusqu'à, au maximum, une valeur limite inférieure, avec des échelons prédéterminés (STEPS), **caractérisé en ce que** la valeur limite inférieure est déterminée en fonction de l'amplitude maximale (PEAK).

2. Détecteur de signal selon la revendication 1, **caractérisé en ce qu'**il est prévu une mémoire d'amplitudes (10B), qui est reliée au dispositif (10A) de détection d'amplitudes et sert à mémoriser l'amplitude maximale du signal d'entrée dans la fenêtre temporelle prédéterminée, et une unité de calcul (15), qui est reliée à la mémoire d'amplitudes et sert à calculer la valeur limite inférieure et/ou supérieure pour la valeur de seuil de détection en fonction de l'amplitude mémorisée, ainsi qu'un premier limiteur (16), qui est relié côté entrée à la sortie de l'unité de calcul (15) et prévu pour la valeur de seuil de détection (TH).

3. Détecteur de signaux selon la revendication 2, **caractérisé en ce qu'**il est prévu un second limiteur (17), qui est branché en série avec le premier limiteur (16) et sert à limiter la valeur de seuil de détection (TH) à une valeur limite absolue prédéterminée de façon fixe.

4. Détecteur de signaux selon l'une des revendications précédentes, **caractérisé en ce qu'**en amont de la sortie est branché un circuit d'arrêt (2, 3, 4), qui sert à bloquer la délivrance d'un autre signal de détection (DETECT) à l'intérieur d'un intervalle de temps prédéterminé (T_{H}) après un signal de détection.

5. Détecteur de signaux selon l'une des revendications précédentes, **caractérisé en ce qu'**au circuit (11, 12A) de commande des paramètres du détecteur est associée une mémoire d'échelons temporels (12B) servant à mémoriser, notamment de façon programmable, au moins une dépendance temporelle, prédéterminée notamment en fonction d'une morphologie de signal connue, de la valeur de seuil de détection.

6. Détecteur de signaux selon l'une des revendications précédentes, **caractérisé en ce qu'**il est prévu une mémoire de valeurs de seuil (10B) et une unité de calcul (18) qui est reliée à la mémoire de valeur de seuil et sert à modifier le paramètre de sensibilité et par conséquent la valeur de seuil de détection, d'un facteur prédéterminé, notamment programmable, ou d'un incrément ou d'un décrément prédéterminé.

7. Détecteur de signaux selon l'une des revendications précédentes, **caractérisé par**
un amplificateur d'entrée (20) comportant une entrée de commande (28) servant à régler un facteur d'amplification,
une mémoire de classification d'amplitude (22) pour mémoriser une valeur de seuil de classification pour établir une distinction entre des événements de détection forts et faibles, et
une unité de comparaison de classification (21) qui est reliée côté entrée à la mémoire de classification d'amplitude (22) et à l'entrée (1a) du détecteur et côté sortie au moins indirectement à l'entrée de commande (20a) de l'amplificateur d'entrée et qui sert à comparer le signal d'entrée à la valeur de seuil de classification mémorisée et à envoyer un signal de commande à l'amplificateur d'entrée lors du dépassement de la valeur de seuil.
